# FASCICULE DE BREVET EUROPEEN

(11) **EP 0 665 828 B1**
(45) Date de publication et mention de la délivrance du brevet: **23.04.1997**
(21) Numéro de dépôt: 93924095.8
(22) Date de dépôt: 19.10.1993
(51) Int. Cl.: C07C 55/14, C07C 51/42, C07C 51/43, C07C 51/14

(54) **PROCEDE D'HYDROXYCARBONYLATION D'ACIDES PENTENOIQUES**
VERFAHREN ZUR HYDROXYCARBOXYLIERUNG DER PENTENSÄUREN
METHOD FOR HYDROXYCARBONYLATING PENTENOIC ACIDS

(30) Priorité: 22.10.1992 FR 9212913
(43) Date de publication de la demande: 09.08.1995
(73) Titulaire: RHONE-POULENC FIBER & RESIN INTERMEDIATES, 92405 Courbevoie (FR)
(72) Inventeur: DENIS, Philippe, F-69150 Décines (FR); KLINGER, François, F-69630 Chaponost (FR); LAURENT, Jean-François, F-69160 Tassin-la-Demi-Lune (FR); PERRON, Robert, F-69390 Charly (FR); SCHWARTZ, Joel, F-69300 Caluire (FR); VACHET, François, F-69150 Décines (FR)
(74) Mandataire: Vignally, Noel
(86) Numéro de dépôt international: FR9301028
(87) Numéro de publication internationale: WO9408939

(56) Documents cités:
- EP-A- 0 188 209
- EP-A- 0 477 112
- WO-A-92/16476
- CA-A- 512 911
- CH-A- 400 116
- US-A- 2 910 516

## Description

La présente invention concerne un procédé d'hydroxycarbonylation des acides penténoïques pour la préparation d'acide adipique, en présence d'un catalyseur qui est au moins en partie séparé du mélange réactionnel obtenu après l'hydroxycarbonylation et réutilisé dans une opération d'hydroxycarbonylation.

Un des procédés les plus prometteurs de préparation de l'acide adipique consiste à hydroxycarbonyler un ou plusieurs acides penténoïques, notamment l'acide pentène-3-oïque et l'acide pentène-4-oïque, à l'aide de monoxyde de carbone et d'eau, en présence d'un catalyseur à base d'iridium, de rhodium ou de leurs mélanges et d'un promoteur iodé. Généralement la réaction est conduite en phase liquide à une température de 50°C à 300°C et sous une pression partielle de monoxyde de carbone de quelques bars. Pour envisager l'utilisation industrielle d'un procédé de ce type, il est bien évidemment indispensable de pouvoir recycler au moins une partie du coûteux catalyseur mis en oeuvre.

La présente invention a précisément pour objet un procédé comportant un tel recyclage.

Parmi les techniques de séparation envisageables, la distillation des composés les plus volatils du mélange réactionnel issu de l'hydroxycarbonylation laisse demeurer le catalyseur avec les diacides formés. Une distillation ultérieure de ces diacides à faible tension de vapeur oblige à les soumettre, ainsi que le catalyseur, à un chauffage prolongé à température relativement élevée, susceptible de les dégrader et de rendre le catalyseur partiellement ou totalement inactif.

La technique de cristallisation nécessite d'assurer un transfert thermique suffisant et de permettre l'obtention de cristaux de taille requise pour être essorés. En outre le taux maximal de solides est limité à une valeur supérieure de l'ordre de 30 à 40 %. Tout cela conduit à un volume d'appareillage important et à l'immobilisation d'une trop grosse quantité de catalyseur très coûteux.

La présente invention propose de résoudre le problème de la séparation et du recyclage du catalyseur dans le cadre d'un procédé d'hydroxycarbonylation d'acides penténoïques en acide adipique, par l'utilisation de la technique du raffinage.

Plus particulièrement, l'invention a pour objet un procédé d'hydroxycarbonylation d'acides penténoïques, caractérisé en ce que:
- l'on hydroxycarbonyle un ou plusieurs acides penténoïques en acide adipique, par le monoxyde de carbone et l'eau, en présence d'un catalyseur à base d'iridium et/ou de rhodium et d'au moins un promoteur iodé,
- le mélange réactionnel obtenu est soumis à une opération de raffinage, après avoir éventuellement été concentré, permettant de séparer au moins une partie du catalyseur,
- le catalyseur ainsi séparé est recyclé dans une opération d'hydroxycarbonylation d'acides penténoïques ou du butadiène ou de dérivés du butadiène.

Par acide penténoïque, on entend dans le cadre de l'invention l'acide pentane-2-oïque, l'acide pentène-3-oïque, l'acide pentène-4-oïque et leurs mélanges.

L'acide pentène-3-oïque pns isolément ou en mélange avec ses isomères est plus particulièrement approprié, en raison de son accessibilité et des résultats satisfaisants auxquels il conduit lors de son hydroxycarbonylation.

Le catalyseur utilisé pour la réaction d'hydroxycarbonylation peut être à base de rhodium, d'iridium ou de ces deux métaux.

N'importe quelle source de rhodium ou d'iridium est susceptible d'être mise en oeuvre.

A titre d'exemples de sources de rhodium pouvant être notamment utilisées, on peut se référer aux composés cités dans le brevet européen EP-A-0 477 112, dont le contenu est incorporé par référence à la présente demande de brevet.

A titre d'exemples de sources d'iridium pouvant être notamment utilisées, on peut citer :
- Ir métallique; Ir O₂ ; Ir₂ O₃ ;
- Ir Cl₃ ; Ir Cl₃,3H₂O ;
- Ir Br₃ ; Ir Br₃,3H₂O ;
- Ir I₃ ;
- Ir₂(CO)₄Cl₂ ; Ir₂(CO)₄I₂ ;
- Ir₂(CO)₈ ; Ir₄(CO)₁₂ ;
- Ir(CO)[P(C₆H₅)₃]₂ I;
- Ir(CO)[P(C₆H₅)₃]₂ Cl ;
- Ir[P(C₆H₅)₃]₃ I ;
- HIr[P(C₆H₅)₃]₃(CO);
- Ir(acac)(CO)₂ ;
- [IrCI(cod)]₂ ;
(cod = cyclooctadiène-1,5 ; acac = acétylacétonate).

Les catalyseurs à base d'iridium qui conviennent plus particulièrement sont : [IrCl(cod)]₂, Ir₄(CO)₁₂ et Ir(acac)(CO)₂.

Les catalyseurs à base d'iridium ou à base d'iridium et de rhodium sont particulièrement préférés dans le cadre du procédé de l'invention.

La quantité de catalyseur à mettre en oeuvre peut varier dans de larges limites.

En général, une quantité exprimée en moles d'iridium métallique et/ou de rhodium métallique par litre de mélange réactionnel comprise entre 10⁻⁴ et 10⁻¹ conduit à des résultats satisfaisants. Des quantités inférieures peuvent être mises en oeuvre: on observe toutefois que la vitesse de réaction est faible. Des quantités supérieures n'ont d'inconvénients qu'au plan économique.

De préférence la concentration en iridium et/ou rhodium est comprise entre 5.10⁻⁴ et 10⁻² mole/litre.

Par promoteur iodé on entend dans le cadre du procédé de l'invention HI et les composés organo-iodés capables de générer HI dans les conditions réactionnelles et plus particulièrement les iodures d'alkyles ayant 1 à 10 atomes de carbone. L'iodure de méthyle est plus particulièrement préconisé.

La quantité de promoteur iodé à mettre en oeuvre est généralement telle que le rapport molaire I/Ir(et/ou Rh) est supérieur ou égal à 0,1. Il n'est pas souhaitable que ce rapport excède 20. De préférence le rapport molaire I/Ir(et/ou Rh) est compris entre 1 et 5.

La présence d'eau est indispensable à la conduite de l'hydroxycarbonylation. Généralement la quantité d'eau à mettre en oeuvre est telle que le rapport molaire eau/acides penténoïques est compris entre 0,01 et 10.

Une quantité supérieure n'est pas souhaitable en raison de la perte d'activité catalytique observée. Le rapport molaire eau/acides penténoïques dans le mélange réactionnel peut être instantanément inférieur à la valeur minimale indiquée précédemment, si l'on procède par exemple à l'injection continue de l'eau, plutôt qu'à son introduction avec les autres charges avant la réaction d'hydroxycarbonylation.

De préférence, le rapport molaire eau/acides penténoïques est compris entre 0,01 et 1, la remarque précédente concernant la valeur minimale étant également valable.

La réaction d'hydroxycarbonylation peut être conduite soit dans un tiers solvant, soit dans un fort excès d'acides penténoïques.

Comme tiers solvant, on peut utiliser notamment les acides carboxyliques aliphatiques saturés ou aromatiques comportant au maximum 20 atomes de carbone pour autant qu'ils soient liquides dans les conditions réactionnelles. A titre d'exemples de tels acides carboxyliques, on peut citer l'acide acétique, l'acide propionique, l'acide butyrique, l'acide valérique, l'acide adipique, l'acide benzoïque et l'acide phénylacétique.

Le tiers solvant peut également être choisi parmi les hydrocarbures saturés aliphatiques ou cycloaliphatiques et leurs dérivés chlorés et les hydrocarbures aromatiques et leurs dérivés chlorés, pour autant que ces composés soient liquides dans les conditions réactionnelles. A titre d'exemples de tels solvants, on peut citer le benzène, le toluène, le chlorobenzène, le dichlorométhane, l'hexane et le cyclohexane.

Lorsqu'il est présent dans le mélange réactionnel, le tiers solvant représente par exemple de 10 % à 99 % en volume par rapport au volume total dudit mélange réactionnel et de préférence de 30 % à 90 % en volume.

Selon une variante préférentielle, la réaction d'hydroxycarbonylation est réalisée dans les acides penténoïques eux-mêmes, c'est-à-dire l'acide pentène-2-oïque, l'acide penténe-3-oïque, l'acide pentène-4-oïque et leurs mélanges.

La réaction d'hydroxycarbonylation est conduite sous une pression supérieure à la pression atmosphérique et en présence de monoxyde de carbone. On peut utiliser le monoxyde de carbone sensiblement pur ou de qualité technique tel qu'il se trouve dans le commerce.

La réaction est conduite en phase liquide. La température est généralement comprise entre 100°C et 240°C et de préférence entre 160°C et 200°C.

La pression totale peut varier dans de larges limites.La pression partielle du monoxyde de carbone, mesurée à 25°C est généralement de 0,5 bar à 50 bars et de préférence de 1 bar à 25 bars.

Le mélange réactionnel issu de la réaction d'hydroxycarbonylation comporte essentiellement les acides penténoïques non transformés, l'eau, le promoteur iodé, le catalyseur, le solvant éventuellement mis en oeuvre, l'acide adipique obtenu, les autres sous-produits formés en quantités plus ou moins importante, comme par exemple l'acide méthyl-2 glutarique, l'acide éthyl-2 succinique, l'acide valérique ou la gamma-valérolactone (ou méthyl-4 butyrolactone).

Ce mélange réactionnel est soumis à une opération de raffinage, soit tel quel, soit de préférence après une opération de concentration, consistant à éliminer, notamment par distillation, une partie ou la totalité du tiers solvant, si un tel solvant a été utilisé, ou une partie de l'excès d'acides penténoïques lorsque la réaction a été conduite sans tiers solvant. Par une telle concentration, on réduit généralement le poids du mélange réactionnel à une valeur représentant de 10 % à 90 % de son poids initial, sans que ces chiffres aient une valeur critique.

Le raffinage dans le présent texte se définit comme la cristallisation directe du mélange réactionnel, de préférence concentré, sur une paroi refroidie, sur laquelle se dépose le raffinat (essentiellement l'acide adipique), tandis que le liquide restant se concentre dans les autres constituants du mélange traité, y compris le catalyseur. Toutefois le réseau cristallin déposé retient par capillarité ou inclusion une quantité limitée du liquide résiduel.

Cette technique est également connue sous l'appellation de cristallisation en milieu fondu.

Dans le cadre du procédé de l'invention, le raffinage a pour principal objectif de séparer la majeure partie de l'acide adipique, du catalyseur qui demeure essentiellement dans le résidu liquide (soutirage); on choisit pour cela un appareil présentant un fort rapport surface d'échange thermique/ volume de mélange réactionnel à traiter, de manière à limiter la durée de cristallisation du raffinat et surtout l'encours de mélange réactionnel et permettre un taux de cristallisation pouvant dépasser 70 %.

L'opération de raffinage peut être décrite de la manière générale suivante.

Le mélange réactionnel à traiter est introduit dans l'appareil, à une température initiale au moins égale à la température de cristallisation du mélange et, de préférence, comprise entre cette température et la température à laquelle a été conduite la réaction d'hydroxycarbonylation; l'appareil est lui-même également à cette température.

Cette température initiale se situe généralement entre 100°C et 200°C.

La première phase de l'opération consiste dans la cristallisation progressive de l'acide adipique sur les parois de l'appareil. Cette cristallisation est obtenue par une diminution programmée de la température des parois d'échange thermique, notamment à l'aide d'un fluide caloporteur circulant de l'autre côté desdites parois.

Selon le type de réalisation industrielle envisagée, la diminution de température peut être plus ou moins rapide, suivant que le mélange à traiter cristallise en couche relativement mince lorsqu'il ne remplit pas tout le volume de l'appareil, ou qu'il cristallise en formant une couche épaisse lorsque le mélange chargé est immobile et remplit tout le volume de l'appareil.

La température finale atteinte est limitée par la température de fusion de l'eutectique des différents constituants du mélange réactionnel. Elle sera choisie en fonction de la composition du mélange réactionnel à raffiner et de la quantité d'acide adipique que l'on souhaite cristalliser. En règle générale, plus cette température sera basse, plus certains autres constituants du mélange réactionnel pourront souiller les cristaux d'acide adipique; à l'inverse, plus cette température sera élevée, plus le taux de récupération de l'acide adipique dans le raffinat sera faible.

En tenant compte de ce qui précède, la température finale est généralement fixée entre 0°C et 70°C.

Lorsque la température finale choisie est atteinte, on récupère, par coulée à la partie inférieure de l'appareillage, la fraction d'égouttage, enrichie en catalyseur et appauvrie en acide adipique par rapport au mélange réactionnel traité. Cela constitue la phase d'égouttage.

La troisième phase de l'opération de raffinage consiste à réaliser un lavage des cristaux déposés sur les parois de l'appareillage, de manière à récupérer la majeure partie du liquide d'égouttage encore retenu sur lesdits cristaux. Elle est réalisée en réchauffant, à une vitesse variable selon le type de réalisation industrielle, les cristaux déposés, ce qui provoque leur fusion partielle; ainsi il se produit un drainage et le remplacement sur les cristaux du liquide d'égouttage par un liquide beaucoup plus riche en acide adipique. Cette phase est appelée phase de ressuage. Son importance quantitative dépend de la quantité de catalyseur que l'on souhaite récupérer et conditionne la pureté et le taux de récupération de l'acide adipique. La fraction de ressuage peut être jointe à la fraction obtenue lors de la phase d'égouttage ou être soumise à une autre opération de séparation de ses constituants par toute technique, notamment par raffinage.

Le terme de soutirage désigne dans le présent texte la partie du mélange réactionnel séparée au cours de la phase d'égouttage et/ou de la phase de ressuage.

La température à laquelle est porté le raffinat pendant la phase de ressuage varie selon la quantité de catalyseur que l'on souhaite récupérer ou la quantité d'acide adipique purifié que l'on désire obtenir.

Cette température se situe généralement entre la température finale de la phase d'égouttage et 150°C. De préférence elle est comprise entre 100°C et 145°C.

Le raffinat déposé sur les parois de l'appareillage peut être ensuite récupéré par une fusion rapide des cristaux.

La durée du cycle de raffinage, constitué par ces différentes phases, peut varier d'environ 1 heure à 30 heures, selon la technologie industrielle adoptée; ainsi un système dynamique à couche mince, offrant une grande surface d'échange thermique par rapport au volume à traiter, nécessitera une durée beaucoup plus réduite qu'un système statique à couche épaisse.

Le soutirage, contenant au moins une partie du catalyseur, du promoteur iodé, des acides penténoïques non transformés ainsi que des quantités plus ou moins importantes des différents constituants du mélange réactionnel traité, peut être directement recyclé dans une réaction d'hydroxycarbonylation (notamment la fraction d'égouttage), ou être préalablement soumis à une ou plusieurs opérations de séparation, visant à séparer le catalyseur de tout ou partie des autres constituants (notamment la fraction de ressuage). Cette séparation supplémentaire peut elle-même être réalisée par la technique de raffinage; mais elle peut être réalisée par toute autre technique conventionnelle, telle que par exemple cristallisation, distillation, extraction.

Le raffinat, contenant en majorité de l'acide adipique ainsi que du catalyseur et des quantités plus ou moins importantes des différents constituants du mélange réactionnel traité, peut être lui-même soumis à une ou plusieurs opérations supplémentaires de séparation, afin de séparer le catalyseur et le promoteur iodé qu'il contient. Comme indiqué précédemment pour le soutirage, cette séparation supplémentaire peut elle-même être réalisée par la technique de raffinage; mais elle peut être réalisée par toute autre technique conventionnelle, telle que par exemple cristallisation, distillation, extraction. Le catalyseur et le promoteur iodé ainsi séparés peuvent alors éventuellement être recyclés.

Le raffinage est conduit de telle façon que l'on sépare au moins 40 % du catalyseur présent dans le mélange réactionnel à traiter et de préférence au moins 80 %.

Un mode préférentiel de l'invention consiste à effectuer une opération de raffinage, au cours de laquelle au moins 80 % du catalyseur sont séparés, entre une température initiale comprise entre 100°C et 200°C et une température finale pour la phase d'égouttage comprise entre 20°C et 60°C, la température étant ensuite remontée entre 100°C et 145°C pour la phase de ressuage; cette opération de raffinage est éventuellement suivie d'une ou plusieurs autres opérations de séparation effectuées sur certaines ou sur toutes les fractions obtenues, pour séparer le reste du catalyseur d'une part et au moins une partie des diacides ramifiés d'autre part. Le catalyseur ainsi obtenu lors du raffinage est recyclé dans la réaction d'hydroxycarbonylation.

Lorsque le catalyseur séparé par l'opération de raffinage est utilisé pour l'hydroxycarbonylation du butadiène ou de ses dérivés, on mettra en oeuvre par exemple la technique décrite pour le rhodium dans les brevets EP-A-0405433 ou EP-A-0274076.

Outre le butadiène, ses dérivés les plus généralement utilisables sont le 3-butène-2-ol, le 2-butène-1-ol, leurs mélanges et leurs esters carboxyliques, notamment les acétates, propionates, valérates, adipates et penténoates.

Les exemples qui suivent illustrent l'invention.

### EXEMPLE 1 - Hydroxycarbonylation de l'acide penténe-3-oïoue

Dans un autoclave muni d'une agitation par turbine auto-aspirante, on introduit:
- 2,45 g (7,3 mmol) d'lr sous forme de [IrCl(cod)]₂ (3,3 mmol/l de mélange réactionnel)
- 4,17 g (18,6 mmol) de HI sous forme d'une solution aqueuse à 57 % en poids par poids (8,4 mmol/l)
- 177,8 g (9,9 mol) d'eau (4,5 mmol/l)
- 2084 g (20,84 mol) d'acide pentène-3-oïque (P3).

L'autoclave, raccordé à l'alimentation de gaz sous pression, est fermé hermétiquement. On admet à froid 2 bar (0,2 MPa) de CO et on chauffe à 185°C en 20 min par l'intermédiaire d'un collier électrique chauffant. Lorsque cette température est atteinte, on régule la pression à 20 bar (2 MPa).

On suit la cinétique de la réaction, par l'absorption de CO dans une réserve reliée au réacteur, la pression dans l'autoclave restant constante.

Après une durée de réaction de 30 min, correspondant à la consommation totale de l'eau introduite, on arrête la réaction en refroidissant le mélange réactionnel. L'autoclave est dégazé et le mélange réactionnel est soutiré à l'état liquide à 120°C.

Le mélange réactionnel est analysé par chromatographie en phase vapeur et par chromatographie liquide haute performance.

On obtient les résultats suivants :
- taux de transformation (TT) de P3 : 51 %
- rendement molaire par rapport à P3 transformé (RT), en acide adipique (A1) : 65 %
- RT en acide méthyl-glutarique (A2) : 11 %
- RT en acide éthyl-succinique (A3) : 3 %
- RT en gamma-valérolactone (VAL) : 11 %
- RT en acide valérique (Pa) : 2 %
- RT en acide pentène-2-oïque (P2) : 8 %

Le taux de linéarité, exprimé par le rapport en pourcentage de A1 formé sur la totalité des diacides A1, A2 et A3 formés, est de 82 %.

La vitesse de réaction (calculée sur 20 min d'absorption de CO) est de 5,4 moles de CO absorbées par heure et par litre de mélange réactionnel.

Le mélange réactionnel est concentré par distillation des produits volatils à 90°C, sous une pression de 1 kPa; on obtient ainsi un mélange que l'on soumet à une opération de raffinage.

### EXEMPLE 2 - Raffinage du mélange réactionnel concentré préparé dans l'exemple 1

L'appareil utilisé est constitué d'un cylindre métallique, de diamètre intérieur 45 mm et de hauteur 65 cm, fermé à sa base par une vanne pneumatique de petit volume et muni à son sommet d'un couvercle vissé traversé par deux sondes de température et une arrivée d'azote.

L'ensemble est entouré d'une double enveloppe permettant la circulation organisée d'un fluide caloporteur à température contrôlée,

On met en oeuvre 1058 g de mélange réactionnel concentré préparé dans l'exemple 1 et ayant la composition pondérale suivante :
- acide adipique 66,00 %
- acide méthyl-2-glutarique 11,20 %
- acide éthyl-2-succinique 2,00 %
- gamma-valérolactone 1,80 %
- acide pentène-2-oïque 3,00 %
- acide pentène-3-oïque 14,50 %
- acide pentène-4-oïque 1,30 %
- iridium (en métal) 0,0887 %
- iode 0,1565 %

Ce mélange réactionnel à l'état liquide à 131°C est chargé dans l'appareil se trouvant à cette même température.

La température est abaissée progressivement à l'aide du caloporteur jusqu'à 40°C en 9 h : c'est la phase de cristallisation.

A 40°C, la vanne de l'appareil est ouverte et la phase d'égouttage s'effectue à température constante pendant 3 h : on récupère ainsi la fraction d'égouttage représentant en poids par poids 20 % du mélange réactionnel chargé.

La température est alors remontée progressivement à 138°C en 11 h ; pendant cette phase de ressuage, on récupère une fraction de ressuage représentant en poids par poids 28 % du mélange réactionnel chargé.

Enfin la température est amenée en 3 heures à la température de fusion du raffinat déposé sur les parois de l'appareil et est maintenue à cette température pendant encore une heure. On récupère ainsi le raffinat qui représente en poids par poids 52 % du mélange réactionnel chargé.

Par dosage par chromatographie én phase gazeuse, par chromatographie liquide haute performance et par fluorescence X, on détermine les compositions respectives de ces trois fractions.

### Fraction d'égouttage :

- acide adipique 10,0 %
- acide méthyl-2-glutarique 32,0 %
- acide éthyl-2-succinique 5,4 %
- gamma-valérolactone 8,4 %
- acide pentène-2-oïque 7,9 %
- acide pentène-3-oïque 33,5 %
- acide pentène-4-oïque 2,5 %
- iridium (en métal) 0,210 %
- iode 0,370 %

### Fraction de ressuage :

- acide adipique 48,0 %
- acide méthyl-2-glutarique 19,5 %
- acide éthyl-2-succinique 3,3 %
- gamma-valérolactone 4,8 %
- acide pentène-2-oïque 5,5 %
- acide pentène-3-oïque 18,5 %
- acide pentène-4-oïque 0 %
- iridium (en métal) 0,133 %
- iode 0,228 %

### Raffinat :

- acide adipique 95,8 %
- acide méthyl-2-glutarique 2,2 %
- acide éthyl-2-succinique 0 %
- gamma-valérolactone 0 %
- acide pentène-2-oïque 0 %
- acide pentène-3-oïque 2,0 %
- acide pentène-4-oïque 0 %
- iridium (en métal) 0,0149 %
- iode 0,0264 %

Le raffinat contient donc 76 % de l'acide adipique et seulement 9 % de l'iridium et de l'iode présents dans le mélange réactionnel issu de la réaction d'hydroxycarbonylation.

La fraction d'égouttage contient 45 % de l'iridium et de l'iode et 3 % de l'acide adipique du mélange réactionnel, tandis que la fraction de ressuage contient 46 % de l'iridium et de l'iode et 21 % de l'acide adipique. Globalement le soutirage constitué par l'ensemble des fractions d'égouttage et de ressuage contient donc 91 % de l'iridium et de l'iode avec 24 % de l'acide adipique du mélange réactionnel. Des partages différents des fractions et du raffinat permettent soit de minimiser la quantité d'acide adipique présent dans le soutirage, soit d'augmenter le taux de récupération de l'iridium et de l'iode selon le critère de séparation privilégié.

### EXEMPLE 3 - Recyclage dans une réaction d'hydroxycarbonylation de la fraction d'égouttage obtenue dans l'exemple 2.

On recycle 100 g de la fraction d'égouttage obtenue dans l'exemple 2. On lui ajoute de l'acide pentène-3-oïque et de l'eau, de manière à avoir un mélange réactionnel initial ayant la même concentration en Ir et en HI que le mélange initial mis en oeuvre dans l'exemple 1, c'est-à-dire 3,3 mmol/l d'lr et 8,5 mmol/l de HI.

Les concentrations du mélange initial pour les autres constituants sont les suivantes :
- 7 mmol/l de P3
- 0,2 mmol/l de P2
- 0,04 mmol/l de P4
- 0,24 mmol/l de VAL
- 0,18 mmol/l de A1
- 0,6 mmol/l de A2
- 0,1 mmol/l de A3
- 4,2 mmol/l d'eau.

L'hydroxycarbonylation est effectuée comme décrit dans l'exemple 1. Après 40 min de réaction, correspondant à la consommation de l'eau introduite, on refroidit l'autoclave et on dose les différents constituants du mélange réactionnel final.

On obtient les résultats suivants :
- taux de transformation (TT) de P3 : 58 %
- RT en acide adipique (A1) : 65 %
- RT en acide méthyl-glutarique (A2) : 13 %
- RT en acide éthyl-succinique (A3) : 3 %
- RT en gamma-valérolactone (VAL) : 9 %
- RT en acide valérique (Pa) : 3 %
- RT en acide pentène-2-oïque (P2) : 7 %

Le taux de linéarité, exprimé par le rapport en pourcentage de A1 formé sur la totalité des diacides A1, A2 et A3 formés, est de 80 %.

La vitesse de réaction (calculée sur 20 min d'absorption de CO) est de 4,4 moles de CO absorbées par heure et par litre de mélange réactionnel. La cinétique étant d'ordre 1 par rapport à P3, on a donc une activité du catalyseur identique à celle observée dans l'exemple 1 (volume absorption de CO/quantité de P3 engagée).

## Revendications

1. Procédé d'hydroxycarbonylation d'acides penténoïques, caractérisé en ce que:
- l'on hydroxycarbonyle un ou plusieurs acides penténoïques en acide adipique par le monoxyde de carbone et l'eau, en présence d'un catalyseur à base d'iridium et/ou de rhodium et d'au moins un promoteur iodé,
- le mélange réactionnel obtenu est soumis à une opération de raffinage, après avoir éventuellement été concentré, permettant de séparer au moins une partie du catalyseur,
- le catalyseur ainsi séparé est recyclé dans une opération d'hydroxycarbonylation d'acides penténoïques.

2. Procédé d'hydroxycarbonylation d'acides penténoïques, caractérisé en ce que:
- l'on hydroxycarbonyle un ou plusieurs acides penténoïques en acide adipique par le monoxyde de carbone et l'eau, en présence d'un catalyseur à base d'iridium et/ou de rhodium et d'au moins un promoteur iodé,
- le mélange réactionnel obtenu est soumis à une opération de raffinage, après avoir éventuellement été concentré, permettant de séparer au moins une partie du catalyseur,
- le catalyseur ainsi séparé est recyclé dans une opération d'hydroxycarbonylation du butadiène et/ou de ses dérivés.

3. Procédé selon l'une des revendications 1 ou 2, caractérisé en ce que l'on sépare par l'opération de raffinage au moins 40 % du poids du catalyseur contenu dans le mélange réactionnel et de préférence au moins 80 %.

4. Procédé selon l'une des revendications 1 ou 2, caractérisé en ce que le mélange réactionnel obtenu après l'opération d'hydroxycarbonylation est concentré avant d'être soumis à l'opération de raffinage pour éliminer au moins une partie des composés les plus volatils qu'il comporte.

5. Procédé selon la revendication 4, caractérisé en ce que le concentrat du mélange réactionnel soumis à l'opération de raffinage représente en poids par poids de 10 % à 90 % du mélange réactionnel issu de l'opération d'hydroxycarbonylation.

6. Procédé selon l'une des revendications 1 à 5, caractérisé en ce que l'hydroxycarbonylation est effectuée dans un solvant choisi parmi les acides carboxyliques tels que les acides aliphatiques saturés et les acides aromatiques ayant moins de 20 atomes de carbone, les hydrocarbures saturés aliphatiques ou cycloaliphatiques et leurs dérivés halogénés, les hydrocarbures aromatiques et leurs dérivés halogénés et les éthers aliphatiques, aromatiques ou mixtes.

7. Procédé selon l'une des revendications 1 à 5, caractérisé en ce que l'hydroxycarbonylation est effectuée dans les acides penténoïques eux-mêmes.

8. Procédé selon l'une des revendications 1 à 7, caractérisé en ce que le raffinage consiste à refroidir de manière contrôlée et progressive le mélange réactionnel,éventuellement concentré, depuis une température initiale au moins égale à la température de cristallisation du mélange et, de préférence, comprise entre cette température et la température à laquelle a été conduite la réaction d'hydroxycarbonylation jusqu'à une température finale supérieure ou égale au point de fusion de l'eutectique des constituants dudit mélange réactionnel, de manière à cristalliser progressivement l'acide adipique du mélange réactionnel.

9. Procédé selon la revendication 8, caractérisé en ce que la température initiale se situe entre 100°C et 200°C et la température finale entre 0°C et 70°C .

10. Procédé selon l'une des revendications 1, 2, 8 et 9, caractérisé en ce que le raffinage comporte une deuxième phase, ou phase d'égouttage, permettant de séparer au moins une partie du catalyseur au sein d'une fraction d'égouttage.

11. Procédé selon l'une des revendications 1, 2, 8 à 10, caractérisé en ce que le raffinage comporte une troisième phase, ou phase de ressuage, consistant, par chauffage progressif des cristaux déposés, à récupérer une fraction de ressuage contenant une autre partie du catalyseur ainsi que d'autres constituants du mélange réactionnel traité.

12. Procédé selon la revendication 11, caractérisé en ce que la phase de ressuage est réalisée par chauffage à une température comprise entre la température finale de la phase d'égouttage et 150°C, et de préférence entre 100°C et 145°C.

13. Procédé selon l'une des revendications 1, 2 et 8 à 12, caractérisé en ce que la durée du cycle de raffinage varie entre 1 heure et 30 heures.

14. Procédé selon l'une des revendications 1, 2 et 8 à 13, caractérisé en ce que la fraction obtenue dans la phase d'égouttage est recyclée dans la réaction d'hydroxycarbonylation.

15. Procédé selon l'une des revendications 1, 2 et 8 à 13, caractérisé en ce que les fractions obtenues dans les phases d'égouttage et de ressuage sont recyclées dans la réaction d'hydroxycarbonylation.

## Patentansprüche

1. Verfahren zur Hydroxycarbonylierung von Pentensäuren, dadurch gekennzeichnet, daß:
- man eine oder mehrere Pentensäuren durch Kohlenmonoxid und Wasser in Gegenwart eines Katalysators auf Iridium- undloder Rhodium-Basis und wenigstens eines iodhaltigen Promotors zu Adipinsäure hydroxycarbonyliert,
- das erhaltene Reaktionsgemisch, nachdem es gegebenenfalls konzentriert wurde, einem Raffinationsvorgang unterzogen wird, der es ermöglicht, wenigstens einen Teil des Katalysators abzutrennen,
- der so abgetrennte Katalysator in einen Vorgang zur Hydroxycarbonylierung von Pentensäuren zurückgeführt wird.

2. Verfahren zur Hydroxycarbonylierung von Pentensäuren, dadurch gekennzeichnet, daß:
- man eine oder mehrere Pentensäuren durch Kohlenmonoxid und Wasser in Gegenwart eines Katalysators auf Iridium- und/oder Rhodium-Basis und wenigstens eines iodhaltigen Promotors zu Adipinsäure hydroxycarbonyliert,
- das erhaltene Reaktionsgemisch, nachdem es gegebenenfalls konzentriert wurde, einem Raffinationsvorgang unterzogen wird, der es ermöglicht, wenigstens einen Teil des Katalysators abzutrennen,
- der so abgetrennte Katalysator in einen Vorgang zur Hydroxycarbonylierung von Butadien und/oder dessen Derivaten zurückgeführt wird.

3. Verfahren gemäß einem der Ansprüche 1 oder 2, dadurch gekennzeichnet, daß man durch den Raffinationsvorgang wenigstens 40% und vorzugsweise wenigstens 80% des Gewichts des in dem Reaktionsgemisch enthaltenen Katalysators abtrennt.

4. Verfahren gemäß einem der Ansprüche 1 oder 2, dadurch gekennzeichnet, daß das nach dem Hydroxycarbonylierungsvorgang erhaltene Reaktionsgemisch, bevor es dem Raffinationsvorgang unterzogen wird, konzentriert wird, um wenigstens einen Teil der flüchtigsten Verbindungen, die es enthält, zu entfernen.

5. Verfahren gemäß Anspruch 4, dadurch gekennzeichnet, daß das dem Raffinationsvorgang unterzogene Konzentrat des Reaktionsgemisches 10 Gew.-% bis 90 Gew.-% des aus dem Hydroxycarbonylierungsvorgang hervorgegangenen Reaktionsgemisches darstellt.

6. Verfahren gemäß einem der Ansprüche 1 bis 5, dadurch gekennzeichnet, daß die Hydroxycarbonylierung in einem Lösungsmittel ausgeführt wird, das unter den Carbonsäuren, wie den gesättigten aliphatischen Säuren und den aromatischen Säuren mit weniger als 20 Kohlenstoffatomen, den aliphatischen oder cycloaliphatischen gesättigten Kohlenwasserstoffen und deren halogenierten Derivaten, den aromatischen Kohlenwasserstoffen und deren halogenierten Derivaten und den aliphatischen, aromatischen oder gemischten Ethern ausgewählt ist.

7. Verfahren gemäß einem der Ansprüche 1 bis 5, dadurch gekennzeichnet, daß die Hydroxycarbonylierung in den Pentensäuren selbst ausgeführt wird.

8. Verfahren gemäß einem der Ansprüche 1 bis 7, dadurch gekennzeichnet, daß die Raffination darin besteht, das gegebenenfalls konzentrierte Reaktionsgemisch kontrolliert und schrittweise von einer Anfangstemperatur, die wenigstens gleich der Kristallisationstemperatur des Gemisches und die vorzugsweise zwischen dieser Temperatur und der Temperatur liegt, bei welcher die Hydroxycarbonylierungsreaktion geführt wurde, bis zu einer Endtemperatur, die höher oder gleich dem Schmelzpunkt des Eutektikums der Bestandteile besagten Reaktionsgemisches ist, abzukühlen, um die Adipinsäure schrittweise aus dem Reaktionsgemisch auszukristallisieren.

9. Verfahren gemäß Anspruch 8, dadurch gekennzeichnet, daß die Anfangstemperatur zwischen 100 °C und 200 °C und die Endtemperatur zwischen 0 °C und 70 °C liegt.

10. Verfahren gemäß einem der Ansprüche 1, 2, 8 und 9, dadurch gekennzeichnet, daß die Raffination eine zweite Phase, oder Abtropfphase, umfaßt, die es ermöglicht, wenigstens einen Teil des Katalysators innerhalb einer Abtropffraktion abzutrennen.

11. Verfahren gemäß einem der Ansprüche 1, 2, 8 bis 10, dadurch gekennzeichnet, daß die Raffination eine dritte Phase, oder Ausschwitzphase, umfaßt, die darin besteht, durch schrittweises Erhitzen der ausgefallenen Kristalle eine Ausschwitzfraktion zu erhalten, die einen anderen Teil des Katalysators sowie andere Bestandteile des behandelten Reaktionsgemisches enthält.

12. Verfahren gemäß Anspruch 11, dadurch gekennzeichnet, daß die Ausschwitzphase durch Erhitzen auf eine Temperatur zwischen der Endtemperatur der Abtropfphase und 150 °C und vorzugsweise zwischen 100 °C und 145 °C durchgeführt wird.

13. Verfahren gemäß einem der Ansprüche 1, 2 und 8 bis 12, dadurch gekennzeichnet, daß die Dauer des Raffinationszyklus zwischen 1 Stunde und 30 Stunden variiert.

14. Verfahren gemäß einem der Ansprüche 1, 2 und 8 bis 13, dadurch gekennzeichnet, daß die in der Abtropfphase erhaltene Fraktion in die Hydroxycarbonylierungsreaktion zurückgeführt wird.

15. Verfahren gemäß einem der Ansprüche 1, 2 und 8 bis 13, dadurch gekennzeichnet, daß die in der Abtropf- und Ausschwitzphase erhaltenen Fraktionen in die Hydroxycarbonylierungsreaktion zurückgeführt werden.

## Claims

1. Process for the hydroxycarbonylation of pentenoic acids, characterized in that:
- one or a number of pentenoic acids are hydroxycarbonylated to adipic acid, by carbon monoxide and water, in the presence of a catalyst based on iridium and/or rhodium and of at least one iodinated promoter,
- the reaction mixture obtained is subjected to a refining operation, after having optionally been concentrated, making it possible to separate at least a part of the catalyst,
- the catalyst thus separated is recycled to an operation for the hydroxycarbonylation of pentenoic acids.

2. Process for the hydroxycarbonylation of pentenoic acids, characterized in that:
- one or a number of pentenoic acids are hydroxycarbonylated to adipic acid, by carbon monoxide and water, in the presence of a catalyst based on iridium and/or rhodium and of at least one iodinated promoter,
- the reaction mixture obtained is subjected to a refining operation, after having optionally been concentrated, making it possible to separate at least a part of the catalyst,
- the catalyst thus separated is recycled to an operation for the hydroxycarbonylation of butadiene and/or of its derivatives.

3. Process according to one of claims 1 or 2, characterized in that at least 40 %, and preferably at least 80 %, of the weight of the catalyst contained in the reaction mixture is separated by the refining operation.

4. Process according to one of claims 1 or 2, characterized in that the reaction mixture obtained after the hydroxycarbonylation operation is concentrated before being subjected to the refining operation in order to eliminate at least a part of the more volatile compounds which it contains.

5. Process according to Claim 4, characterized in that the concentrate of the reaction mixture subjected to the refining operation represents, in weight by weight, from 10 % to 90 % of the reaction mixture emerging from the hydroxycarbonylation operation.

6. Process according to one of claims 1 to 5, characterized in that the hydroxycarbonylation is carried out in a solvent chosen from carboxylic acids such as saturated aliphatic acids and aromatic acids having less than 20 carbon atoms, saturated aliphatic or cycloaliphatic hydrocarbons and their halogenated derivatives, aromatic hydrocarbons and their halogenated derivatives and aliphatic, aromatic or mixed ethers.

7. Process according to one of claims 1 to 5, characterized in that the hydroxycarbonylation is carried out in the pentenoic acids themselves.

8. Process according to one of claims 1 to 7, characterized in that the refining consists in cooling the optionally concentrated reaction mixture in a controlled and progressive way from an initial temperature at least equal to the crystallization temperature of the mixture, and preferably between this temperature and the temperature at which the hydroxycarbonylation reaction was carried out, to a final temperature greater than or equal to the melting point of the eutectic of the constituents of the said reaction mixture, so as progressively to crystallize the adipic acid of the reaction mixture.

9. Process according to claim 8, characterized in that the initial temperature is between 100°C and 200°C and the final temperature between 0°C and 70°C.

10. Process according to one of claims 1, 2, 8 and 9, characterized in that the refining contains a second phase, or draining phase, making it possible to separate at least a part of the catalyst within a draining fraction.

11. Process according to one of claims 1, 2, 8 to 10, characterized in that the refining contains a third phase, or sweating phase, consisting, by progressive heating of the deposited crystals, in recovering a sweating fraction containing another part of the catalyst and other constituents of the treated reaction mixture.

12. Process according to claim 11, characterized in that the sweating phase is carried out by heating at a temperature between the final temperature of the draining phase and 150°C, and preferably between 100°C and 145°C.

13. Process according to one of claims 1, 2 and 8 to 12, characterized in that the duration of the refining cycle varies between 1 hour and 30 hours.

14. Process according to one of claims 1, 2 and 8 to 13, characterized in that the fraction obtained in the draining phase is recycled to the hydroxycarbonylation reaction.

15. Process according to one of claims 1, 2 and 8 to 13, characterized in that the fractions obtained in the draining and sweating phases are recycled to the hydroxycarbonylation reaction.
